# EUROPEAN PATENT APPLICATION

(11) **EP 1 610 250 A1**
(43) Date of publication of application: **28.12.2005**
(21) Application number: 05013324.8
(22) Date of filing: 21.06.2005
(51) Int. Cl.: G06F 17/60

(54) **System for facilitating weight control incorporating hand-held computing device**

(30) Priority: 21.06.2004 US 872880
(71) Applicant: Weightwatchers.com., Inc., New York, NY 10106 (US)
(72) Inventor: Basone, Michael A., New Canaan, Connecticut 06840 (US); Laginestra, Michael, Southport, Connecticut 06890 (US); McKee, Joseph T., New York, New York 10023 (US); Huang, Sharon S. H., Maplewood, New Jersey 07040 (US); Kelly, Sheila, New York 10003 (US); Parlee, Scott, New Jersey 07043 (US); Filkin, Donna, Caldwell, New Jersey 07006 (US); Fiarman, Jeffrey A., Ridgewood, New Jersey 07450 (US)
(74) Representative: Brötz, Helmut

(57) **Abstract**

A system and method for facilitating weight control provides dieters with the ability to maintain food consumption within the general rules of a weight control program and to maintain motivation in following these rules, incorporates a hand-held computing device to facilitate use of the system, provides the ability to update at least some information on either a server portion of the system or the hand-held computing device and then to synchronize the pertinent information therebetween at a later time, does not rely solely upon costly and often unreliable wireless communications, is not cumbersome to use, and is conducive to keeping information up-to-date.

## Description

### Field of the Invention

The present invention generally relates to a system for facilitating weight control, and more specifically, to a software and hardware system operable to facilitate weight control which includes a hand-held computing device, such as a personal digital assistant (PDA).

### Background of the Invention

People have long sought ways to control (i.e., lose, gain, and/or maintain) body weight. Controlling body weight has many implications to peoples' lives, including: physical health, mental health, and professional and social status. Entire industries have been formed to assist individuals who seek assistance in weight control. Such industries include medical care, food production, publishing, workout facilities, and support groups, to name a few.

Weight control programs (e.g., diets) to control body weight have been developed by many individuals, groups, and organizations and disseminated throughout the different industries. Some of the weight control programs may be considered proprietary and others are publicly available. For example, medical organizations may provide patients with weight control programs that have been developed for patients, but not made publicly available otherwise. Developers of weight control programs earn money by making the programs publicly available through various sources, including books, videos, lectures and tapes, for example. Of course, the rules of weight control programs and the food types that they prescribe are all different, and the diversity of different weight control programs throughout the weight loss industry is considerable: diets range from those ordered by a physician as part of a treatment for a disease or clinical condition to calorie-counting diets, vegetarian diets, protein rich diets, low carbohydrate diets, sodium gram diets, fluid-restricted diets, renal diets (which utilize fluid, protein and specific electrolyte restrictions such as sodium, potassium, etc.) and cardiac diets (which utilize specific fat, salt, and cholesterol restrictions).

No matter which of the various sources a developer of a weight control program utilizes to make its weight control program publicly available or which type of diet the weight control program utilizes, it is the responsibility of the individual following the weight control program to adhere to the rules, guidelines, and conventions (general rules) of the weight control program. These rules may define the specific foods, times to eat and exercises to be performed, for example. As most individuals who have followed a weight control program can testify, in addition to maintaining self-motivation, learning and following these often complex rules tend to be the most difficult parts of staying on, and achieving success with, a weight control program. What is desirable, therefore, is a way to provide dieters with the ability to maintain food consumption within the general rules of a weight control program and to maintain motivation in following these rules.

To overcome the problems of having to follow the complex general rules of a weight control program and to promote self-motivation to adhere to such a weight control program, numerous software and hardware systems have been developed that allows users to actively participate in following a weight control program. Many of such systems have been implemented on stand-alone computer systems (e.g., personal computers) or via a networked system of computers (e.g., via the Internet). While such systems possess a number of desirable attributes, they suffer from a number of disadvantages. One of such disadvantages relates to the fact that the persons using such systems often need access to the systems while away from their computers, such as for example when eating, shopping, exercising, etc. outside the home or workplace (or wherever their computers are located). This is true even of so-called lap-top computers, which while being portable, are often too cumbersome to carry around on one's person wherever access to weight control software may be needed.

In order to obviate this problem, several weight control systems have been developed which employ hand-held computing devices, such as personal digital assistants (PDAs). U.S. Patent No. US 6,478,736 B1 and U.S. Patent Application Publication No. US 2002/0133378 A1 to Mault disclose integrated calorie management systems which incorporate a portable electronic device (disclosed in some embodiments as comprising a PDA) to which a meter for measuring a resting metabolic rate of the user is connected. The resting metabolic rate, as recorded by the portable electronic device is used by the system for weight control purposes. U.S. Patent Application Publication No. US 2002/0072932 A1 discloses a PDA having a health care program running thereon, which program does include some diet management functionality.

In both systems, it is disclosed that the PDA may be in communication with Internet websites (i.e., servers), such being desirable so that updates to the software executing on the PDA, and databases stored on the PDA, can be made from the server as the databases are updated on the server, and so that information concerning foods consumed (among other information) may be transmitted from the PDA to the server (and later accessed by, for example, the user's physician). It is disclosed that the manner in which the PDA communicates with the server may be either (i) through a direct wireless link from the PDA to the server, or (ii) by a two stage synching operation, where during one operation information between the server and a personal computer are synchronized, and during another separate operation (when the PDA is docked with the personal computer) information between the personal computer and the PDA are synchronized.

While these systems do provide advantages over previously known systems, they do still suffer from a number of disadvantages of their own. For example, while the disclosed communications between the PDA and the servers is bi-directional (in that information flows both ways via the communications link), there is no bi-directional updating of the same type of information. More specifically, in the systems disclosed in the above references, when the foods/exercises database is updated on the server, the corresponding database on the PDA is appropriately updated during the synchronizing operation. However, in the prior art systems, there is no way for the foods/exercises database to be updated on the PDA, and then for the corresponding database on the server to be updated during the synchronizing procedure. Similarly, when the information concerning foods consumed is updated on the PDA, the corresponding information on the server is appropriately updated during the synchronizing operation. However, in the prior art systems, there is no way for the foods consumed information to be updated on the server, and then for the corresponding information on the PDA to be updated during the synchronizing procedure.

This disadvantageously limits the flexibility to the user. For example, it may be desirable for the user to update the foods/exercises database on the PDA while he/she is purchasing items at the grocery store and then to upload the changes to the server at a later time, rather than being required to update the database on the server and then download the changes to the PDA. Similarly, it may be desirable for the user to update information, such as information concerning foods consumed or exercises performed, directly on the server, for example, if the user does not have ready access to the PDA, and then to later download the information to the PDA. What is desirable, therefore, is the ability to update at least some information on either the server or the PDA and then to synchronize the pertinent information to the other device at a later time, rather than being required to enter only certain types of information on the PDA and other types of information on the server.

Another disadvantage of the prior art systems disclosed in the above references relates to the manner in which communications between the PDA and the server is achieved. As mentioned above, It is disclosed that the manner in which the PDA communicates with the server may be either (i) through a direct wireless link from the PDA to the server, or (ii) by a two stage synching operation, where during one operation information between the server and a personal computer are synchronized, and during another separate operation (when the PDA is docked with the personal computer) information between the personal computer and the PDA are synchronized. The first of these methods of communication is disadvantageous for several reasons. First, such wireless communications may be costly, requiring relatively costly hardware and access fees for wireless air time. Moreover, such wireless communications may not be readily available, particularly in non-urban environments. The second of the methods of communication is disadvantageous in that it is cumbersome, requiring multiple operations, and may not be conducive to keeping information up-to-date, for example, if the user only performs one-half of the two-step synchronizing operation on a regular basis.

What is desired, therefore, is a system for facilitating weight control which provide dieters with the ability to maintain food consumption within the general rules of a weight control program and to maintain motivation in following these rules, which incorporates a hand-held computing device to facilitate use of the system, which provides the ability to update at least some information on either a server portion of the system or the hand-held computing device and then to synchronize the pertinent information therebetween at a later time, which does not rely solely upon costly and often unreliable wireless communications, which is not cumbersome to use, and which is conducive to keeping information up-to-date.

### Summary of the Invention

Accordingly, it is an object of the present invention to provide a system for facilitating weight control which provide dieters with the ability to maintain food consumption within the general rules of a weight control program and to maintain motivation in following these rules.

Another object of the present invention is to provide a system for facilitating weight control having the above characteristics and which incorporates a hand-held computing device to facilitate use of the system.

A further object of the present invention is to provide a system for facilitating weight control having the above characteristics and which provides the ability to update at least some information on either a server portion of the system or the hand-held computing device and then to synchronize the pertinent information therebetween at a later time.

Still another object of the present invention is to provide a system for facilitating weight control having the above characteristics and which does not rely solely upon costly and often unreliable wireless communications.

Yet a further object of the present invention is to provide a system for facilitating weight control having the above characteristics and which is not cumbersome to use.

Still another object of the present invention is to provide a system for facilitating weight control having the above characteristics and which is conducive to keeping information up-to-date.

These and other objects of the present invention are achieved according to one embodiment of the present invention by provision of a system for facilitating weight control of a user including a hand-held computing device having weight control software executing thereon and having a handheld database with weight control data stored thereon, the weight control data being used by the weight control software during execution thereof. The system also includes a personal computing device, a first communications link by which the hand-held computing device and the personal computing device are communicable during a synchronization operation, a server having a server database with weight control data stored thereon, and a second communications link by which the personal computing device and the server are communicable during the synchronization operation. During the synchronization operation, weight control data or modifications thereto are transferred in substantially real time between the handheld database and the server database. At least a portion of the weight control data comprises two-way data modifiable by the user both on the handheld database via the hand-held computing device and on the server database via the server. When the two-way data is modified by the user on the handheld database via the hand-held computing device the system automatically makes corresponding modifications to the weight control data stored on the server database during the synchronization operation, and when the two-way data is modified by the user on the server database via the server the system automatically makes corresponding modifications to the weight control data stored on the handheld database during the synchronization operation.

In some embodiments, the system further includes middleware software for facilitating the synchronization operation executing on at least one of the hand-held computing device, the personal computing device and the server. In certain of these embodiments, the middleware software for facilitating the synchronization operation is executing on all three of the hand-held computing device, the personal computing device and the server. In some embodiments, the server comprises a middleware server having executing thereon middleware software for facilitating the synchronization operation and a website server. In certain of these embodiments, the middleware server and the website server comprise a single computing device.

In some embodiments, the hand-held computing device comprises at least one of a personal digital assistant (PDA), a hand-held personal computer and a wireless telephone. In some embodiments, the first communications link comprises a handheld cradle connected to the personal computing device. In some embodiments, the second communications link comprises at least one of the Internet, a satellite communications network, a wireless or wired telecommunications network, a local area network (LAN) and a wide area network (WAN).

In some embodiments, at least a portion of the weight control data comprises one-way data modifiable by the user only on the server database via the server and when the one-way data is modified by the user on the server database via the server the system automatically makes corresponding modifications to the weight control data stored on the handheld database during the synchronization operation. In certain of these embodiments, the one-way data comprises at least one of foods data, manufacturers data, categories data, companion data, portions and portions type data, activities data, index data and basic user information data. In some embodiments, the two-way data comprises at least one of meal entries data, journal entries data and favorites data.

In some embodiments, conflicts between the two-way data stored on the server database and the two-way data stored on the handheld database are resolved during synchronization. In certain embodiments, entries of the two-way data include time and date stamps, and the conflicts are resolved such that entries are modified if data contained in subsequent entries is different than data contained in earlier entries. In certain embodiments, entries of the two-way data include date stamps, and the conflicts are resolved on a calendar day basis. In certain of these embodiments, the user specifies which of the two-way data stored on the server database and the two-way data stored on the handheld database has priority during conflict resolution.

In accordance with another embodiment of the present invention, a system for facilitating weight control of a user includes a hand-held computing device having weight control software executing thereon and having a handheld database with weight control data stored thereon, the weight control data being used by the weight control software during execution thereof. The system also includes a server having a server database with weight control data stored thereon and a communications link by which the hand-held computing device and the server are communicable. At least a portion of the weight control data comprises two-way data modifiable by the user both on the handheld database via the hand-held computing device and on the server database via the server. When the two-way data is modified by the user on the handheld database via the hand-held computing device the system automatically makes corresponding modifications to the weight control data stored on the server database during a synchronization operation, and when the two-way data is modified by the user on the server database via the server the system automatically makes corresponding modifications to the weight control data stored on the handheld database during the synchronization operation.

In some of these embodiments, the communications link comprises a personal computing device, a first communications link by which the hand-held computing device and the personal computing device are communicable during the synchronization operation, and a second communications link by which the personal computing device and the server are communicable during the synchronization operation. In these embodiments, during the synchronization operation, the two-way weight control data or modifications thereto are transferred in substantially real time between the handheld database and the server database.

In accordance with another embodiment of the present invention, a system for facilitating weight control of a user includes a hand-held computing device having weight control software executing thereon and having a handheld database with weight control data stored thereon, the weight control data being used by the weight control software during execution thereof. The system also includes a personal computing device, a first communications link by which the hand-held computing device and the personal computing device are communicable during a synchronization operation, a server having a server database with weight control data stored thereon, and a second communications link by which the personal computing device and the server are communicable during the synchronization operation. During the synchronization operation, weight control data or modifications thereto are transferred in substantially real time between the handheld database and the server database.

In some of these embodiments, at least a portion of the weight control data comprises two-way data modifiable by the user both on the handheld database via the hand-held computing device and on the server database via the server. In these embodiments, when the two-way data is modified by the user on the handheld database via the hand-held computing device the system automatically makes corresponding modifications to the weight control data stored on the server database during the synchronization operation, and when the two-way data is modified by the user on the server database via the server the system automatically makes corresponding modifications to the weight control data stored on the handheld database during the synchronization operation.

In another respect, the present invention is concerned with methods of operation of systems for facilitating weight control of a user as described above.

The invention and its particular features and advantages will become more apparent from the following detailed description considered with reference to the accompanying drawings.

### Brief Description of the Drawings

Figure 1 is a schematic diagram illustrative of the interaction between users and a provider of a weight control software system in accordance with an embodiment of the present invention;

Figure 2 is a schematic diagram illustrating in more detail the weight control software system of Figure 1;

Figure 3 is a block diagram illustrating operation of the weight control software system of Figure 1;

Figure 4 is a block diagram illustrating in more detail than Figure 3 operation of the weight control software system of Figure 1;

Figures 5a-5c are block diagrams illustrating operation of a handheld computing device aspect of the weight control software system of Figure 1; and

Figure 6 is a block diagram illustrating a synchronization operation of the handheld computing device aspect shown in Figures 5a-5c.

### Detailed Description of an Embodiment of the Invention

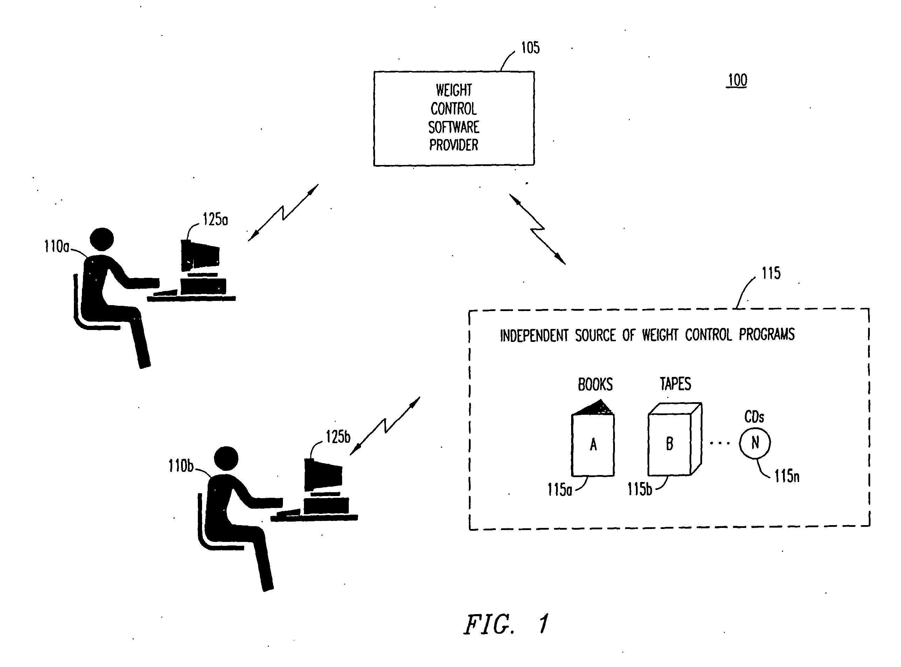

Figure 1 is an exemplary diagram illustrative of (i) interaction between a weight control software provider 105 and users 110a-110b, (collectively 110), where the weight control software provider 105 provides the general rules and other elements of any weight control program 115a-115n (collectively 115) via a weight control software system, and (ii) interaction among (a) users 110, (b) an external source that provides users with the general rules and other elements of the weight control program 115, and (c) the weight control software provider 105 that provides the weight control software system, in each case according to the principles of the present invention. The weight control software system is accessed via the computing systems 125a or 125b (collectively 125) by the users 110a or 110b, respectively.

The weight control program 115 may be any dietary system or technique that allows the user 110 to lose, maintain, or gain body weight. The weight control program 115 may be designed and developed by any entity (including the user 110 him/herself) and may have different rules, guidelines and conventions. These general rules may be provided to the user 110 either directly by the weight control software system via downloading over a network or through external sources, such as, but without limitation, books, tapes, lectures or CD-ROMs.

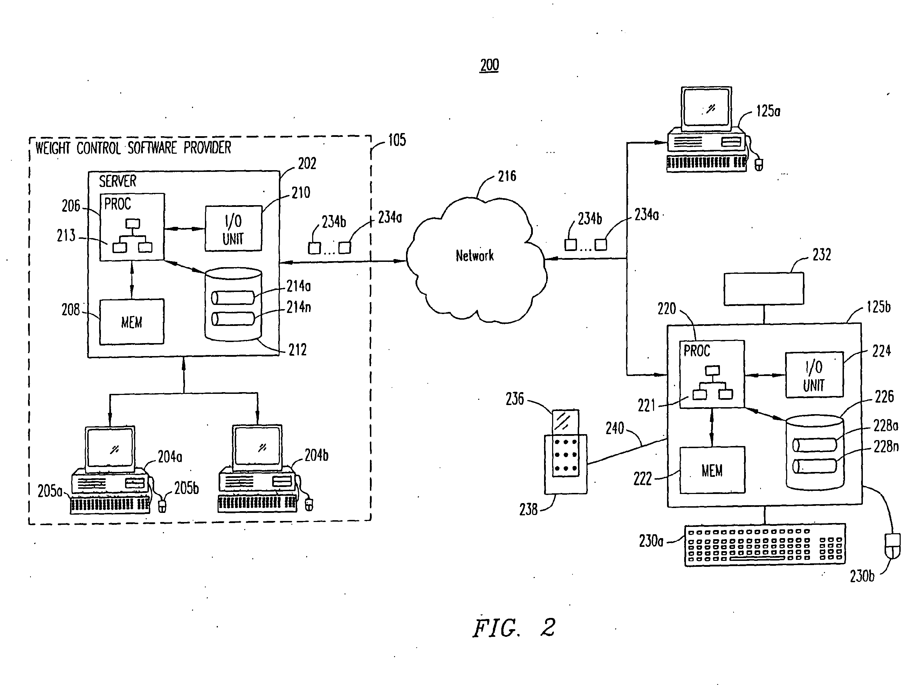

Figure 2 is an exemplary block diagram 200 for operating a weight control software system for the weight control software provider 105 and users 110 of Figure 1. The weight control software provider 105 utilizes a server 202 for managing and maintaining the weight control software system or a portion thereof (e.g., databases may be located externally from the server 202).

As is understood in the art, remote terminals 204a and 204b (collectively 204) are operable by the weight control software provider 105 to interact with the server 202 to maintain the weight control software system. The server 202 includes a processor 206 coupled to a memory 208. The processor 206 is further coupled to an input/output (I/O) unit 210 and storage device 212. The storage device 212 may store one or more server database 214a-214n that include data associated with the weight control software system provided by the weight control software provider 105. Server software 213 is operable to maintain and distribute data composed as datasets associated with individual users 110 of the weight control software system.

The server 202 is coupled to a network 216. The network 216 may be any network, for example, the Internet, a satellite communications network, a wireless or wired telecommunications network, local area network (LAN), wide area network (WAN), or any combination thereof. Additionally, the computing systems 125 utilized by the users 110 are coupled to the network 216. As shown, the computing system 125b includes a processor 220 operating software 221 coupled to a memory 222. The software 221 may include an interface (e.g., a web browser) as understood in the art and facilitate interface and execution with the server software 213 for the user 110 to utilize the weight control software system. The weight control software system may provide for a weight control program 115 (Figure 1) by storing rules of one or more weight control program 115 on the storage device 212. The weight control software system may thereafter read and logically follow the rules of the weight control program 115 as understood in the art. The processor 220 is further coupled to an I/O unit 224 (e.g., a modem) and a storage device 226. The storage device 226 may store user databases 228a-228n (collectively 228), where the user databases 228 may include data that is a subset of the server databases 214.

The computing system 125b further includes input control devices 230a and 230b, such as a keyboard and computer mouse, for operating the weight control software system. A display 232 is also coupled to the computing system 125b for display of information provided by the weight control software system. While the computing systems 125 are shown as desktop computing systems, it should be understood that laptop, other configured computing systems, or terminals (e.g., interactive televisions) may be utilized.

In operation, the users 110 utilize the computing system 125 for executing and utilizing the weight control software system. As is understood in the art, the user 110 using the software 221 and associated hardware (e.g., I/O unit 224) may connect to the server 202 via the network 216. Data packets 234a and 234b (collectively 234) are utilized to communicate data of the weight control software system across the network 216 from the server 202 to the computing systems 125 and vise versa. The server 202 may host a website that supports the weight control program 115 (Figure 1) and provide access to the user 110. The data communicated across the network 216 may include web pages and weight control data stored in the server databases 214 to the computing systems 125 for storage or utilization thereby.

The web pages may be displayed on the display 232, and utilize the data stored in the user databases 228 to allow the user 110 to monitor and maintain the weight control data associated with the weight control program 115. The network interaction between the user 110 and the weight control software provider 105 provides the users 110 with a means for interactively and dynamically adhering to the weight control program 115.

A hand-held computing device 236 may communicate with the computing system 125b by a cradle 238 coupled via a wire 240 and may operate the weight control software system independent of or in communication with the network 216. In other embodiments, the hand-held computing device may communicate directly with the server 202 via the network 216 or another communications link. The communications between the hand-held computing device 236 and the computing system 125b and/or the server 202 are described in more detail below. The hand-held computing device may be a personal digital assistant, hand-held personal computer, wireless telephone, or other electronic device capable of executing the weight control software system or a reduced version derived therefrom. The hand-held computing device 236 may be synchronized with the information from the computing system 125b as described in more detail below. Accordingly, the hand-held computing device 236 may be capable of downloading data of the user 110, updating the data, and uploading the data for use and/or storage and communication by the computing system 125b.

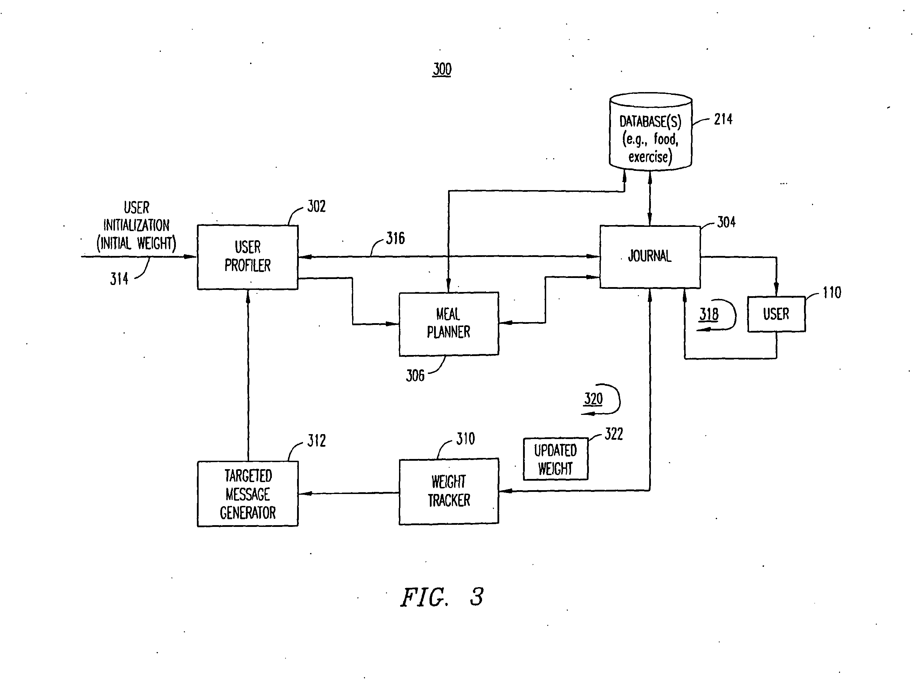

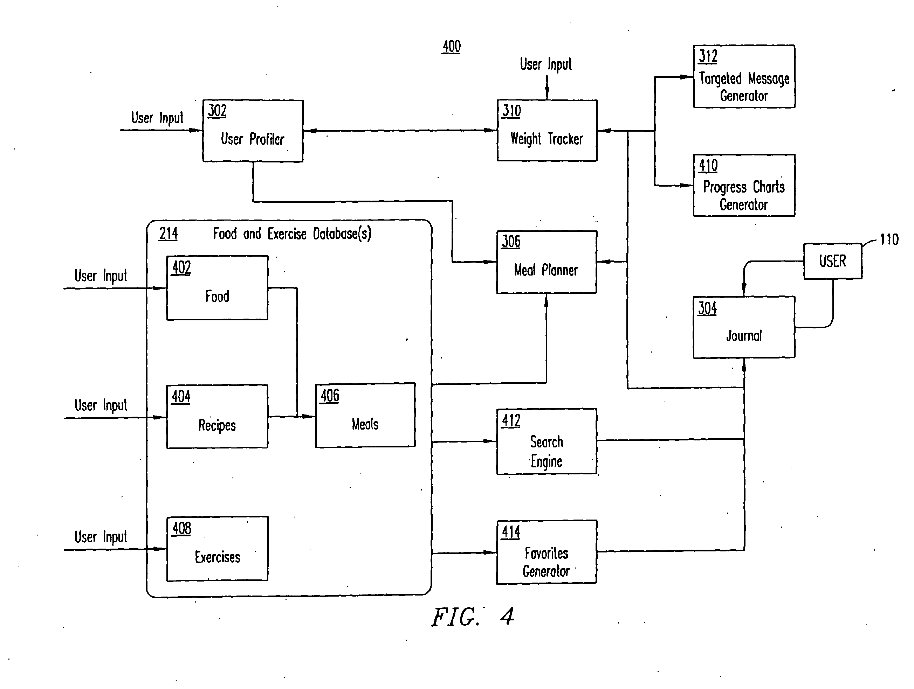

Figures 3 and 4 are exemplary block diagrams that model the process, whereby the weight control software system utilizes personal data that is input by the user 110 to (i) customize the weight control software system, (ii) provide the users 110 with individualized feedback, and/or (iii) update the interactive software environment and underlying architecture. The weight control software system manages personal data for a given user 110 that is stored on the server 202. As users 110 enter personal information into the user profiler 302 and subsequently update or include additional information in the weight control software system, the user 110 individualizes the weight control software system and updates certain tools and modules of the weight control software system according to such personal information. The customized weight control software system further aids in the control of body weight by providing feedback to users 110 based on their individual progress utilizing the applicable weight control program 115.

As shown in the block diagram 300 a number of components of the model representative of the operation of the weight control software system are configured in an inter-relational manner so as to provide the user 110 with personalization and feedback capabilities. As shown, a user profiler 302 is interconnected directly to a journal 304 and interconnected via a meal planner 306. The server databases 214 may be interconnected to both the journal 304 and meal planner 306 so as to more globally provide access to the data or information stored in the server databases 214. A weight tracker 310 is interconnected to the journal 304 and additionally interconnected to a targeted message generator 312. The targeted message generator 312 may further be interconnected to the user profiler 302. The user profiler 302, journal 304, meal planner 306, server databases 214, and weight tracker 310 may provide the user 110 with the capability of utilizing and maintaining data provided or pre-established by the weight control software provider 105 (Figure 1) and user-entered data.

Again referring to Figure 3, the weight control software system utilizes personal information entered by the user 110 to (i) customize the weight control software system, (ii) provide the users 110 with individualized feedback, and/or (iii) update the interactive software environment and underlying architecture. As shown, two feedback loops 318 and 320 are provided in the block diagram 300. Feedback loop 318 may be considered a periodic (e.g., daily) loop that is generated as the user 110 works with the journal 304. Alternatively, the feedback loop 318 may be considered event driven as the weight control software system is utilized by the user 110. The journal 304 provides a constant reminder and motivator for the user 110 to maintain, manage, and adhere to the weight control program 115 (Figure 1). In other words, the user 110 is provided daily feedback and reminders by simply working with the journal 304 and the information provided therein.

Although the journal 304 and meal planner 306 are shown to be coupled, the components of the weight control software system may be provided to the user 110 by the weight control software system individually and independently. By allowing the components to operate individually and independently, the user 110 may be provided a more limited scope of functionality, but have suitable functionality for the purposes desired by the user 110.

Feedback loop 320 may be considered a periodic or aperiodic feedback loop that is formed by the user 110 performing a weigh-in, where the weigh-in measures the current or updated weight 322 of the user 110. The updated weight 322 may be supplied by the user 110 to the weight tracker 310, which utilizes the updated weight 322 to determine the progress of the user 110.

Figure 4 is a more detailed exemplary block diagram 400 of underlying architecture components of the weight control software system of Figure 3. As shown, the components (e.g., journal, databases, meal planner) of the weight control software system are modular and interoperable. In other words, the information provided to one of the components is accessible to each of the other components.

The server databases 214 have been expanded to show a number of different databases, including food 402, recipes 404, meals 406 and exercises or activities 408. Each of these databases may include pre-established data provided by the weight control product provider 105 (Figure 1) and user-entered data provided by the user 110. The food database 402 may include food served by restaurants, such as McDonald's® and other brand name restaurants and food products. In addition to the targeted message generator 312, the user 110 is able to utilize a progress chart generator 410 to monitor parameters and/or performance indicators that are indicative of the progress of the user 110 in following the weight control program. For example, the progress chart generator 410 may receive updated weights from the weight tracker 310 and display the updated weights over a period of time so that the user 110 can monitor weight loss, for example. By graphically monitoring or feeding-back weight loss progress, the user 110 may find additional motivation. The graphical representation may additionally allow the user 110 to identify successful periods (e.g., weeks) of weight loss so that the user 110 may review the journal 304 to determine what meals made those periods successful.

Two additional components that are included in the more detailed block diagram 400 of the weight control software system are a search engine 412 and a favorites generator 414. The search engine 412 allows the user 110 to search the server databases 214 for particular words and/or food values. The search engine 412 may be more comprehensive and allow the user 110 to search for types of foods, courses, or any other information that may be stored in the server databases 214 as understood in the art. The favorites generator 414 allows the user 110 to identify and categorize individual foods, meals, recipes, and/or exercises that the user 110 often uses. By including both of these components, the user 110 is able to save time in utilizing the weight control software system. Additionally, the user 110 is able to further customize the weight control software system according to personal desires.

The weight control software system possesses a high level of interoperability and interconnection within the interactive software environment and underlying architecture (including the modules and tools) such that user input in one module or tool may update other modules and tools for increased user efficiency and personalization.

Referring again to FIG. 3, the journal 304 operates as a blank piece of virtual paper that the user 110 personalizes by recording food and exercise consumption in performing the personal data management, and enables the weight control software system to provide feedback as to when the user 110 is adhering to the weight control program 115 (Figure 1). The journal 304 provides the user 110 with access to a calendar (not shown) that lists the meals and/or foods eaten for each present and/or past day. Meal plans generated by the meal planner 306 may also may be automatically and/or semi-automatically posted to the journal 304.

The user 110 may enter food and exercise into the journal 304 by searching the server databases 214 via the search engine 412. The search engine 412 allows the user 110 to search the server databases 214 for particular words and/or food metrics based on the general rules of the weight control program 115 (Figure 1). The search engine 412 may be more comprehensive and allow the user 110 to search for types of foods, courses, or any other information that may be stored in the server databases 214 as understood in the art. Any food or exercise entered into the journal 304 by the user 110 may be saved by the server databases 214 as a separate user-customized and defined "favorites" category. The favorites generator 414 allows the user 110 to identify and categorize individual foods, meals, recipes, and/or exercises that the user 110 often uses, thus allowing the user 110 to save time while utilizing the weight control software system.

The journal 304 allows the user 110 to post and record consumed foods in a calendar format established in conjunction with the weight control program 115 supported by the weight control software system. And, the meals and/or foods consumed by the user 110 may be stored by the journal 304 for future reference purposes by the user 110 or weight control software system.

In addition to individual foods, recipes and exercises, the user 110 may access through the meal planner 306 certain predetermined meal plans developed in accordance with the general rules of the weight control program and stored on the server databases 214. The meal planner 306 determines meals for each user 110 in accordance with the personal profile of the user 110 and the general rules of any weight control program 115.

If the user 110 does not want to use a meal provided by the meal planner 306, the user 110 may replace this meal with an alternative meal generated by the meal planner 306 and consistent with the general rules of the weight control program 115. The user 110 may input any meal generated by the meal planner 306 into the journal 304 and save the meal in the favorites category of the user 110. Furthermore, as the weight tracker 310 is updated by the updated weight 322 as input by the user 110, the meal planner 306 automatically alters the dietary recommendations of the weight control program 115 (Figure 1) based on the updated weight 322 in accordance with the general rules of the weight control program 115. By altering the dietary recommendations of the weight control program 115, the recommended quantity and type of food is altered such that both the journal 304 settings and the meals provided to the user 110 via the meal planner 306 are automatically updated.

The weight tracker 310 utilizes the general rules of the weight control program 115 to maintain the information of the user 110. The weight tracker 310 is operable to automatically alter the recommended amount or type of food a user 110 should consume based on the updated weight 322 into the weight tracker 310 and the general rules of the weight control program 115. In addition, the meals provided to the user 110 via the meal planner 306 are automatically altered. The user profiler 302 may also be updated with the updated weight 322.

As the user 110 loses weight, the weight tracker 310 may reduce the amount or type of food that the user 110 is recommended to consume because as the user 110 loses weight, fewer nutrients are required by the user 110. The recommendation may be based on a body mass index (BMI), cholesterol levels, body fat measurements, etc., and lowered using a linear or non-linear technique. It should be understood that the weight control program 115 may additionally be utilized to assist a user 110 in gaining weight. In the case of gaining weight, the recommended amount or type of food may be set higher such that the weight control program 115 operates to increase the body mass index of the user 110.

The general rules of the weight control program 115 may range from simple to complex and may be based on any number of criteria, such as food items, calories, nutrients, weight measurements, and exercise levels. In one embodiment, the rules of the weight control program 115 prescribe that the user 110 is to lose not more than a maximum number of pounds over a certain amount of time. As understood in the art, by maintaining a steady loss of weight, the user 110 is provided a safe way to lose weight. Other rules may be applied for safety or medical reasons as established for a specific user based on the profile or otherwise.

Upon or after entry of the updated weight 322, a targeted message may be delivered by the targeted message generator 312 in an instantaneous or real-time, or substantially real-time fashion such that the user 110 is provided feedback based on the updated weight 322. The feedback in the form of the targeted message may be instantaneous. Alternatively the targeted message may be delayed. The targeted message generator 312 may issue an instant message, e-mail, and/or customized web page, for example. The targeted message may include a congratulatory statement, encouragement statement, motivational statement, or other statement or content made to the user 110 based on the updated weight 322. In other words, if the user 110 loses weight from the previous week, then the targeted message generator 312 may congratulate the user on his or her accomplishment.

Since the loss of a few pounds for one individual may be relatively insignificant relative to his or her ultimate weight goal, the targeted message may be adjusted based on the goals set by the user 110. By providing instantaneous feedback to the user 110, instant gratification or satisfaction may be provided to the user 110 to help encourage and motivate the user 110 to maintain use of the weight control program 115 and use the weight control software system. The targeted message may also provide a warning if the user is losing weight too quickly. In addition, the targeted message may be tailored to the perceived success or failure of the user 110 with his or her weight loss by asking the user 110 questions about his or her weight loss or gain prior to delivering the targeted message. The targeted message may be just what the user 110 needs to provide that added recognition to maintain a healthy attitude on the journey to his or her ultimate weight goal.

The weight tracker 310 updates the progress chart generator 410 to monitor parameters and/or performance indicators that are indicative of the progress of the user 110 in following the weight control program 115. For example, the progress chart generator 410 may receive updated weights 322 from the weight tracker 310 and display the updated weights over a period of time so that the user 110 can monitor weight loss or gain, for example. By graphically monitoring or feeding-back weight loss progress, the user 110 may be additionally motivated. The graphical representation may additionally allow the user 110 to identify successful weeks of weight loss so that the user 110 may review the journal 304 to determine what meals made those weeks successful.

It should be noted that much of the functionality described above in connection with Figures 1-4 is described in co-pending U.S. Patent Application Serial No. 10/355,425 which is commonly owned with the present application, and the entirety of which is incorporated by reference herein.

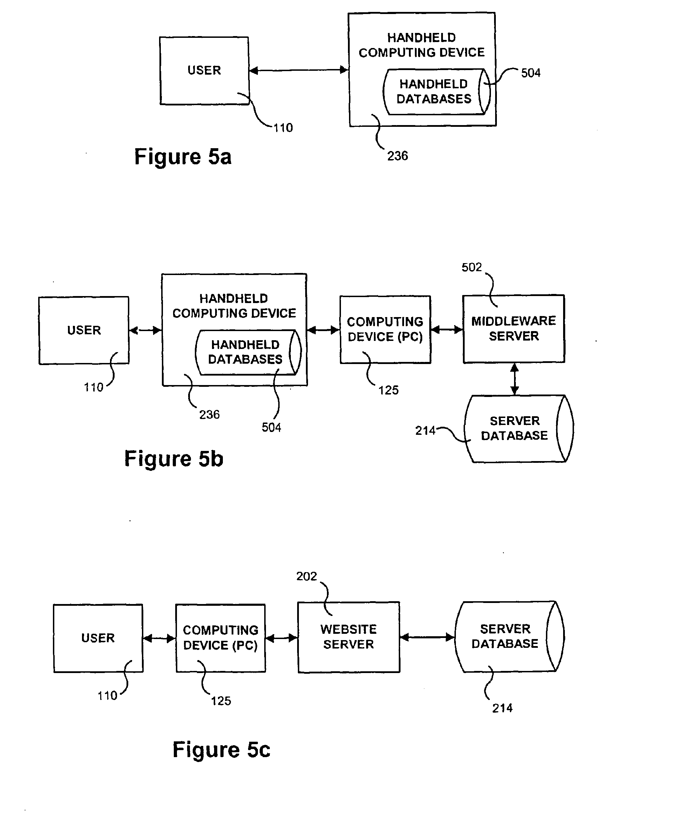

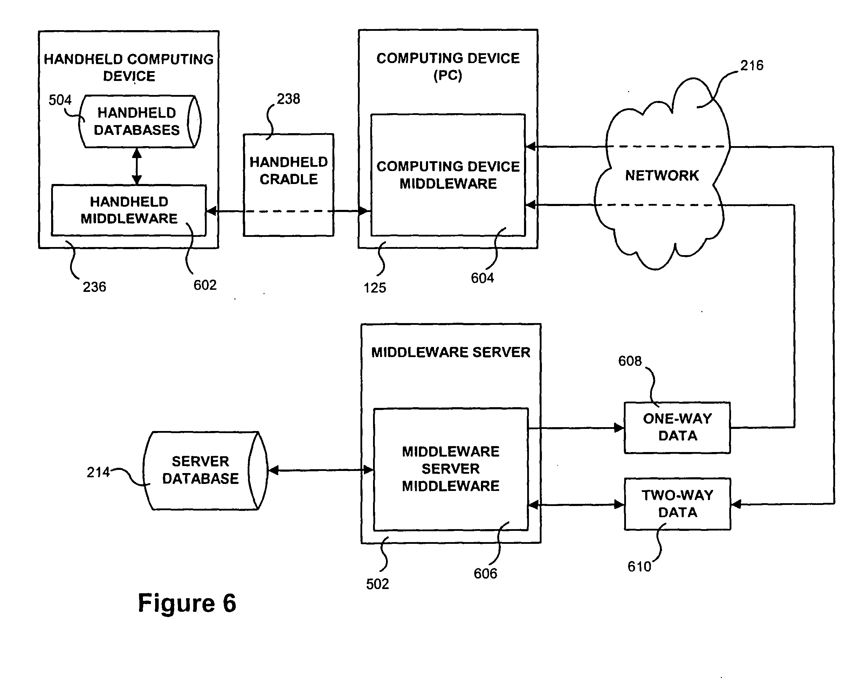

Referring now to Figures 5a-5c and Figure 6, the present invention provides much of the functionality of weight control program 115 (Figure 1) on hand-held computing device 236. The end user of the system is a customer able to use an application on his/her hand-held computing device to manage his/her weight loss program. The PDA service, further allows this user's PDA to exchange data securely with the central server 202 infrastructure. This integrated full-system functionality means that for the end user the PDA and the website are both viable ways to use the weight control program 115, and that data is shared between both media.

The overall PDA solution architecture is conceptually illustrated in Figures 5a-5c. The architecture can be logically described as a client-server system, where the client consists of a suite of on-device applications and the server consists of a database infrastructure and related business middleware. The general system model is that a user 110 interacts with either the website via computing device 125 and website servers 202 (Figure 5c), or his/her handheld application executing on hand-held computing device 236, entering and managing his/her journal data with the help of associated central databases 202 of foods, exercises, meals, recipes, etc. (Figure 4). Changes made by a specific user in one medium are easily propagated to the other medium as explained in more detail below. For example, meal entries created by a user on his/her handheld and stored on handheld databases 504 will be delivered to the server databases 214 so that they also become visible via the website, and vice-versa.

While the changes made via the website are instantaneously (to the user) stored in the backend, changes made via the PDA application are made in an offline or disconnected mode. These offline changes are then propagated to the backend (i.e., website servers 214) via computing device 125 and middleware server 502 through a process referred to as synchronization (Figure 5b). The exchange of data between the two media, through this synchronization process, is now described.

Most of the functionality involved in synchronizing changes to on-device handheld databases 504 (made by the handheld application) with the server database 214 used by the website is handled by middleware software designed for this purpose. Preferably, this software offers general data mapping services, including conflict resolution support, between server database 214 and handheld databases 504.

This middleware software consists of both a client and server component. The server is deployed as a production service within the weight control service provider 105 operational infrastructure. The client is distributed to handheld devices 236. The handheld application, with user input, interacts with the on-device handheld databases 504, and the middleware handles moving these changes securely and robustly to the server infrastructure via user-initiated synchronizations. The reverse process takes place for changes propagated to the client from the server. At a high level, this process can be thought of as replicating data between copies of the core databases that exist on the handheld device and on the server. The purposes of the middleware can be summarized as follows: (1) Providing an over-the-Internet link between user handhelds (attached to a networked desktop via a cradle) and the server infrastructure; (2) Managing all data security, authentication, and access control between client and server; (3) Mapping data between the handheld application and server software, formatting and translating appropriately for each platform; and (4) Providing logic points for conflict resolution and other business rules.

It is important to note the directionality of changes. Specifically, some data is replicated exclusively from server (single-master replication) to client. This data is read-only to the end user of the handheld application - examples include the core food database. In other scenarios, data is in fact replicated in both directions. This is the case specifically with the journal functionality - changes to the journal are made on the handheld as well as on the site. In this scenario, conflict resolution becomes a necessary part of the middleware equation. Further details concerning this aspect of the invention are discussed below.

In addition to the core middleware layer itself, there are numerous utilities which are preferably provided to support the operation of the overall client-server system. For example, the handheld application is provided with a version of the core weight control program data to be available on the device. The handheld application introduces a substantial indexing scheme to allow this large quantity of data to be navigated (searched) efficiently and effectively. The generation of this core data and its related indices is left to an out of band process, such that the only runtime requirements on the system are that the handheld device be able to obtain the more or less static data files for read-only use. In other words, for data that is more or less static and requires substantial indexing, this data can be prepared out of band and the middleware can simply be used to do traditional binary file downloads.

At the heart of the PDA system is, as at the heart of the website itself, the server-side databases that house all the general and user-specific data for the solution. These databases represent the core of the system. The PDA solution, through the middleware, delivers this data to individual handhelds. The architecture by which this delivery occurs actually involves the use of a decoupled PDA solution-specific database, such that the middleware and therefore the rest of the PDA system do not directly touch the core site databases. This decoupling offers several specific advantages, but at the highest level it is done to isolate the site from performance or functional ramifications introduced by the PDA solution.

The "database infrastructure" of the PDA solution, then, consists of a PDA-specific database containing all data that needs to be delivered to and exchanged with the handheld platform (via the middleware). A replication process exists to ensure that the data in the PDA-specific database is refreshed in both directions with the core site databases. The salient design point here is that the synchronization with the handheld is asynchronously decoupled from the actual database used by the site.

Generally as used herein, what is meant by "core" data is data which is common to all users. This data is read-only, and is relatively static (updated quarterly). This data may include, for example, data concerning foods, manufacturers, categories, companions, portions and portion types, activities and indexes supporting fast navigation of these large datasets. There is also a set of user-specific data which, given functionality of the handheld application, is read-only on the handheld. This data is dynamic and is therefore synchronized in one direction (server to client) as often as necessary. This data may include basic member information. Together these two types of data (i.e., the core data and the read-only user specific data) are generally referred to as "one-way" data. In addition, there is some data, such as the journal data, meal entries data and favorites data (recipes, meals, foods, activities, etc.), which is member-specific and read-write on the client application. Since this data requires bi-directional synchronization (with appropriate conflict resolution), this data is referred to as "two-way" data.

Referring now to Figure 6, the synchronization aspect of the present invention is shown in greater detail. The user initiates the synchronization operation in a usual manner, such as by docking handheld computing device 236 in a handheld cradle 238, or by in some other way placing handheld 236 in communication with computing device 125. During such synchronization, handheld middleware 602 residing on handheld 236 is in communication, via handheld cradle 238 with computing device middleware 604 residing on computing device 125. As part of this same operation (i.e., at the same time), communication is established between computing device middleware 604 and middleware server middleware 606 residing on middleware server 502 via network connection 216. As such, handheld middleware 602 is in substantially real-time communication with middleware server middleware 606. With handheld middleware 602 being able to access and update data stored on handheld databases 504, and with middleware server middleware 606 being able to access and update data stored on server database 214, data on handheld databases 504 can be synchronized in substantially real time with data on server database 214, rather then requiring two separate synchronization operations.

With respect to some data which is intended to be read-only on handheld computing device 236, data and/or updates to such data (i.e., one-way data 608) are transferred in only one direction from server database 214 to handheld databases 504. This one-way transfer of data is appropriate for data which can not be modified by the user on handheld 236 -- because such data can not be modified on handheld, there would never be any reason to transfer data or updates to such data from handheld databases 504 to server database 214. However, as discussed above, there are certain types of data which can be modified by the user or by others on both the handheld databases 504 and/or on the server database 214. With respect to this data (i.e., two-way data 610) the data transfer during synchronization occurs in two directions, from server database 214 to handheld databases 504, and at the same time from handheld databases 504 to server database 214. This allows for at least certain types of data to be modified on either the handheld or on the website at the user's convenience.

As will be recognized by those skilled in the art, by allowing at least some data (i.e., two-way data 610) to be transferred during synchronization in two directions, from server database 214 to handheld databases 504, and at the same time from handheld databases 504 to server database 214, there exists the possibility for conflicts to occur between the data stored on each database. A conflict occurs when a change (addition, deletion or modification) is made to an entry on both the handheld databases 504 and the server database 214.

There are several options for dealing with such conflicts. In one conflict resolution scheme, each entry includes a true time stamp (i.e., one indicating date as well as time of day). While there are multiple possible methods for dealing with conflicts, one preferred method operates such that entries are modified if data contained in subsequent entries is different than data contained in earlier entries.

In accordance with a simple example of such a conflict resolution scheme suppose that a user enters breakfast data on the server database 214, lunch data on the handheld databases 504, and dinner data on the server database 214, and then performs a synchronization operation. All data (i.e., breakfast data, lunch data and dinner data) would appear in both places (i.e., on both server database 214 and handheld databases 504).

A somewhat more complex example is now presented: For breakfast, if the user entered eggs on the handheld databases 504 and bacon on the server database 214, both would appear in both places after synchronization. If the user entered two fried eggs on the handheld databases 504 and one fried egg on the server database 214, both would show three fried eggs. If the user entered two fried eggs on the server database 214 and then performed a synchronization operation, and then on the handheld databases 504 deleted the eggs and entered pancakes and performed another synchronization operation, pancakes would appear in both places but not eggs.

Another, more simple, conflict resolution scheme is now presented, in which the system stores only a date stamp (as opposed to both a date stamp and a time stamp). In such cases, conflicts may be resolved on the basis of a calendar day, rather than on the basis of date and time of day. Conflicts may be resolved in favor of handheld databases 504 or the server database 214. In some embodiments, the user may specify which databases have priority. So, for example, assuming that conflicts are resolved in favor of the server database 214, if a user enters breakfast data on the server database 214, lunch data on the handheld databases 504, and dinner data on the server database 214, and then performs a synchronization operation, the lunch data will be lost. The data would not be lost if the user performs synchronization operations after breakfast and again after lunch, since only supplemental data, and no conflicting data, would be perceived. Another example: If the user enters Tuesday's meals on the handheld databases 504 and Wednesday's meals on the server database 214 and then performs a synchronization operation, there will be no conflict since the differing data is for two different calendar days.

The present invention, therefore, provides a system for facilitating weight control which provide dieters with the ability to maintain food consumption within the general rules of a weight control program and to maintain motivation in following these rules, which incorporates a hand-held computing device to facilitate use of the system, which provides the ability to update at least some information on either a server portion of the system or the hand-held computing device and then to synchronize the pertinent information therebetween at a later time, which does not rely solely upon costly and often unreliable wireless communications, which is not cumbersome to use, and which is conducive to keeping information up-to-date.

Although the invention has been described with reference to a particular arrangement of parts, features and the like, these are not intended to exhaust all possible arrangements or features, and indeed many other modifications and variations will be ascertainable to those of skill in the art.

## Claims

1. A system for facilitating weight control of a user, said system comprising:
a hand-held computing device having weight control software executing thereon and having a handheld database with weight control data stored thereon, the weight control data being used by the weight control software during execution thereof;
a personal computing device;
a first communications link by which said hand-held computing device and said personal computing device are communicable during a synchronization operation;
a server having a server database with weight control data stored thereon;
a second communications link by which said personal computing device and said server are communicable during the synchronization operation;
wherein during the synchronization operation, weight control data or modifications thereto are transferred in substantially real time between the handheld database and the server database; and
wherein at least a portion of the weight control data comprises two-way data modifiable by the user both on the handheld database via the hand-held computing device and on the server database via the server, wherein when the two-way data is modified by the user on the handheld database via said hand-held computing device said system automatically makes corresponding modifications to the weight control data stored on the server database during the synchronization operation, and wherein when the two-way data is modified by the user on the server database via said server said system automatically makes corresponding modifications to the weight control data stored on the handheld database during the synchronization operation.

2. The system of Claim 1 further comprising middleware software for facilitating the synchronization operation executing on at least one of said hand-held computing device, said personal computing device and said server.

3. The system of Claim 2 further comprising middleware software for facilitating the synchronization operation executing on all three of said hand-held computing device, said personal computing device and said server.

4. The system of Claim 1 wherein said server comprises a middleware server having executing thereon middleware software for facilitating the synchronization operation and a website server.

5. The system of Claim 4 wherein the middleware server and the website server comprise a single computing device.

6. The system of Claim 1 wherein said hand-held computing device comprises at least one of a personal digital assistant (PDA), a hand-held personal computer and a wireless telephone.

7. The system of Claim 1 wherein said first communications link comprises a handheld cradle connected to said personal computing device.

8. The system of Claim 1 wherein said second communications link comprises at least one of the Internet, a satellite communications network, a wireless or wired telecommunications network, a local area network (LAN) and a wide area network (WAN).

9. The system of Claim 1 wherein at least a portion of the weight control data comprises one-way data modifiable by the user only on the server database via the server and wherein when the one-way data is modified by the user on the server database via said server said system automatically makes corresponding modifications to the weight control data stored on the handheld database during the synchronization operation.

10. The system of Claim 9 wherein the one-way data comprises at least one of foods data, manufacturers data, categories data, companion data, portions and portions type data, activities data, index data and basic user information data.

11. The system of Claim 1 wherein the two-way data comprises at least one of meal entries data, journal entries data and favorites data.

12. The system of Claim 1 wherein conflicts between the two-way data stored on the server database and the two-way data stored on the handheld database are resolved during synchronization.

13. The system of Claim 12 wherein entries of the two-way data include time and date stamps, and wherein the conflicts are resolved such that entries are modified if data contained in subsequent entries is different than data contained in earlier entries.

14. The system of Claim 12 wherein entries of the two-way data include date stamps, and wherein the conflicts are resolved on a calendar day basis.

15. The system of Claim 14 wherein the user specifies which of the two-way data stored on the server database and the two-way data stored on the handheld database has priority during conflict resolution.

16. A system for facilitating weight control of a user, said system comprising:
a hand-held computing device having weight control software executing thereon and having a handheld database with weight control data stored thereon, the weight control data being used by the weight control software during execution thereof;
a server having a server database with weight control data stored thereon;
a communications link by which said hand-held computing device and said server are communicable; and
wherein at least a portion of the weight control data comprises two-way data modifiable by the user both on the handheld database via the hand-held computing device and on the server database via the server, wherein when the two-way data is modified by the user on the handheld database via said hand-held computing device said system automatically makes corresponding modifications to the weight control data stored on the server database during a synchronization operation, and wherein when the two-way data is modified by the user on the server database via said server said system automatically makes corresponding modifications to the weight control data stored on the handheld database during the synchronization operation.

17. The system of Claim 16 wherein at least a portion of the weight control data comprises one-way data modifiable by the user only on the server database via the server and wherein when the one-way data is modified by the user on the server database via said server said system automatically makes corresponding modifications to the weight control data stored on the handheld database during the synchronization operation.

18. The system of Claim 17 wherein the one-way data comprises at least one of foods data, manufacturers data, categories data, companion data, portions and portions type data, activities data, index data and basic user information data.

19. The system of Claim 16 wherein the two-way data comprises at least one of meal entries data, journal entries data and favorites data.

20. The system of Claim 16 wherein said hand-held computing device comprises at least one of a personal digital assistant (PDA), a hand-held personal computer and a wireless telephone.

21. The system of Claim 16 wherein said communications link comprises at least one of the Internet, a satellite communications network, a wireless or wired telecommunications network, a local area network (LAN) and a wide area network (WAN).

22. The system of Claim 16 wherein said communications link comprises:
a personal computing device;
a first communications link by which said hand-held computing device and said personal computing device are communicable during the synchronization operation;
a second communications link by which said personal computing device and said server are communicable during the synchronization operation; and
wherein during the synchronization operation, the two-way weight control data or modifications thereto are transferred in substantially real time between the handheld database and the server database.

23. The system of Claim 16 wherein conflicts between the two-way data stored on the server database and the two-way data stored on the handheld database are resolved during synchronization.

24. The system of Claim 23 wherein entries of the two-way data include time and date stamps, and wherein the conflicts are resolved such that entries are modified if data contained in subsequent entries is different than data contained in earlier entries.

25. The system of Claim 23 wherein entries of the two-way data include date stamps, and wherein the conflicts are resolved on a calendar day basis.

26. The system of Claim 25 wherein the user specifies which of the two-way data stored on the server database and the two-way data stored on the handheld database has priority during conflict resolution.

27. A system for facilitating weight control of a user, said system comprising:
a hand-held computing device having weight control software executing thereon and having a handheld database with weight control data stored thereon, the weight control data being used by the weight control software during execution thereof;
a personal computing device;
a first communications link by which said hand-held computing device and said personal computing device are communicable during a synchronization operation;
a server having a server database with weight control data stored thereon;
a second communications link by which said personal computing device and said server are communicable during the synchronization operation; and
wherein during the synchronization operation, weight control data or modifications thereto are transferred in substantially real time between the handheld database and the server database.

28. The system of Claim 27 further comprising middleware software for facilitating the synchronization operation executing on at least one of said hand-held computing device, said personal computing device and said server.

29. The system of Claim 28 further comprising middleware software for facilitating the synchronization operation executing on all three of said hand-held computing device, said personal computing device and said server.

30. The system of Claim 27 wherein said server comprises a middleware server having executing thereon middleware software for facilitating the synchronization operation and a website server.

31. The system of Claim 30 wherein the middleware server and the website server comprise a single computing device.

32. The system of Claim 27 wherein said hand-held computing device comprises at least one of a personal digital assistant (PDA), a hand-held personal computer and a wireless telephone.

33. The system of Claim 27 wherein said first communications link comprises a handheld cradle connected to said personal computing device.

34. The system of Claim 27 wherein said second communications link comprises at least one of the Internet, a satellite communications network, a wireless or wired telecommunications network, a local area network (LAN) and a wide area network (WAN).

35. The system of Claim 27 wherein at least a portion of the weight control data comprises two-way data modifiable by the user both on the handheld database via the hand-held computing device and on the server database via the server, wherein when the two-way data is modified by the user on the handheld database via said hand-held computing device said system automatically makes corresponding modifications to the weight control data stored on the server database during the synchronization operation, and
wherein when the two-way data is modified by the user on the server database via said server said system automatically makes corresponding modifications to the weight control data stored on the handheld database during the synchronization operation.

36. A method for facilitating weight control of a user, said method comprising the steps of:
providing a hand-held computing device having weight control software executing thereon and having a handheld database with weight control data stored thereon, the weight control data being used by the weight control software during execution thereof;
providing a personal computing device;
establishing a first communications link by which the hand-held computing device and the personal computing device are communicable during a synchronization operation;
providing a server having a server database with weight control data stored thereon;
establishing a second communications link by which the personal computing device and the server are communicable during the synchronization operation;
performing a synchronization operation and transferring in substantially real-time weight control data or modifications thereto between the handheld database and the server database;
wherein at least a portion of the weight control data comprises two-way data modifiable by the user both on the handheld database via the hand-held computing device and on the server database via the server; and
receiving modifications of the two-way data on the handheld database from the user via the hand-held computing device and automatically making corresponding modifications to the weight control data stored on the server database, or receiving modifications of the two-way data on the server database from the user via the server and automatically making corresponding modifications to the weight control data stored on the handheld database.

37. The method of Claim 36 further comprising the step of facilitating the synchronization operation by providing middleware software executing on at least one of the hand-held computing device, the personal computing device and the server.

38. The method of Claim 37 further comprising the step of facilitating the synchronization operation by providing middleware software executing on all three of the hand-held computing device, the personal computing device and the server.

39. The method of Claim 36 wherein the server comprises a middleware server and a website server and further comprising the step of facilitating the synchronization operation by providing middleware software executing on the middleware server.

40. The method of Claim 39 wherein the middleware server and the website server comprise a single computing device.

41. The method of Claim 36 wherein the hand-held computing device comprises at least one of a personal digital assistant (PDA), a hand-held personal computer and a wireless telephone.

42. The method of Claim 36 wherein the first communications link comprises a handheld cradle connected to the personal computing device.

43. The method of Claim 36 wherein the second communications link comprises at least one of the Internet, a satellite communications network, a wireless or wired telecommunications network, a local area network (LAN) and a wide area network (WAN).

44. The method of Claim 36 wherein at least a portion of the weight control data comprises one-way data modifiable by the user only on the server database via the server and further comprising the step of receiving modifications to the one-way data on the server database by the user via the server and automatically making corresponding modifications to the weight control data stored on the handheld database.

45. The method of Claim 44 wherein the one-way data comprises at least one of foods data, manufacturers data, categories data, companion data, portions and portions type data, activities data, index data and basic user information data.

46. The method of Claim 36 wherein the two-way data comprises at least one of meal entries data, journal entries data and favorites data.

47. The method of Claim 36 further comprising the step of resolving conflicts between the two-way data stored on the server database and the two-way data stored on the handheld database during synchronization.

48. The method of Claim 47 further comprising the step of time and date stamping entries of the two-way data, and wherein the conflicts are resolved such that entries are modified if data contained in subsequent entries is different than data contained in earlier entries.

49. The method of Claim 47 further comprising the step of date-stamping entries of the two-way data, and wherein the conflicts are resolved on a calendar day basis.

50. The method of Claim 49 further comprising the step of receiving from the user a specification of which of the two-way data stored on the server database and the two-way data stored on the handheld database has priority during conflict resolution.

51. A method for facilitating weight control of a user, the method comprising the steps of:
providing a hand-held computing device having weight control software executing thereon and having a handheld database with weight control data stored thereon, the weight control data being used by the weight control software during execution thereof;
providing a server having a server database with weight control data stored thereon;
establishing a communications link by which the hand-held computing device and the server are communicable; and
wherein at least a portion of the weight control data comprises two-way data modifiable by the user both on the handheld database via the hand-held computing device and on the server database via the server; and
receiving modifications of the two-way data on the handheld database from the user via the hand-held computing device and automatically making corresponding modifications to the weight control data stored on the server database, or receiving modifications of the two-way data on the server database from the user via the server and automatically making corresponding modifications to the weight control data stored on the handheld database.

52. The method of Claim 51 wherein at least a portion of the weight control data comprises one-way data modifiable by the user only on the server database via the server and further comprising the step of receiving modifications to the one-way data on the server database by the user via the server and automatically making corresponding modifications to the weight control data stored on the handheld database.

53. The method of Claim 52 wherein the one-way data comprises at least one of foods data, manufacturers data, categories data, companion data, portions and portions type data, activities data, index data and basic user information data.

54. The method of Claim 51 wherein the two-way data comprises at least one of meal entries data, journal entries data and favorites data.

55. The method of Claim 51 wherein the hand-held computing device comprises at least one of a personal digital assistant (PDA), a hand-held personal computer and a wireless telephone.

56. The method of Claim 52 wherein the communications link comprises at least one of the Internet, a satellite communications network, a wireless or wired telecommunications network, a local area network (LAN) and a wide area network (WAN).

57. The method of Claim 51 wherein said step of establishing a communications link comprises the steps of:
providing a personal computing device;
establishing a first communications link by which the hand-held computing device and the personal computing device are communicable during the synchronization operation;
establishing a second communications link by which the personal computing device and the server are communicable during the synchronization operation; and
performing a synchronization operation and transferring in substantially real-time weight control data or modifications thereto between the handheld database and the server database.

58. The method of Claim 51 further comprising the step of resolving conflicts between the two-way data stored on the server database and the two-way data stored on the handheld database during synchronization.

59. The method of Claim 58 further comprising the step of time and date stamping entries of the two-way data, and wherein the conflicts are resolved such that entries are modified if data contained in subsequent entries is different than data contained in earlier entries.

60. The method of Claim 58 further comprising the step of date-stamping entries of the two-way data, and wherein the conflicts are resolved on a calendar day basis.

61. The method of Claim 60 further comprising the step of receiving from the user a specification of which of the two-way data stored on the server database and the two-way data stored on the handheld database has priority during conflict resolution.

62. A method for facilitating weight control of a user, the method comprising:
providing a hand-held computing device having weight control software executing thereon and having a handheld database with weight control data stored thereon, the weight control data being used by the weight control software during execution thereof;
providing a personal computing device;
establishing a first communications link by which the hand-held computing device and the personal computing device are communicable during a synchronization operation;
providing a server having a server database with weight control data stored thereon;
establishing a second communications link by which the personal computing device and the server are communicable during the synchronization operation; and
performing a synchronization operation and transferring in substantially real-time weight control data or modifications thereto between the handheld database and the server database.

63. The method of Claim 62 further comprising the step of facilitating the synchronization operation by providing middleware software executing on at least one of the hand-held computing device, the personal computing device and the server.

64. The method of Claim 63 further comprising the step of facilitating the synchronization operation by providing middleware software executing on all three of the hand-held computing device, the personal computing device and the server.

65. The method of Claim 63 wherein the server comprises a middleware server and a website server and further comprising the step of facilitating the synchronization operation by providing middleware software executing on the middleware server.

66. The method of Claim 65 wherein the middleware server and the website server comprise a single computing device.

67. The method of Claim 62 wherein the hand-held computing device comprises at least one of a personal digital assistant (PDA), a hand-held personal computer and a wireless telephone.

68. The method of Claim 62 wherein the first communications link comprises a handheld cradle connected to the personal computing device.

69. The method of Claim 62 wherein the second communications link comprises at least one of the Internet, a satellite communications network, a wireless or wired telecommunications network, a local area network (LAN) and a wide area network (WAN).

70. The method of Claim 62 wherein at least a portion of the weight control data comprises two-way data modifiable by the user both on the handheld database via the hand-held computing device and on the server database via the server, and further comprising the step of receiving modifications of the two-way data on the handheld database from the user via the hand-held computing device and automatically making corresponding modifications to the weight control data stored on the server database, or receiving modifications of the two-way data on the server database from the user via the server and automatically making corresponding modifications to the weight control data stored on the handheld database.
